# FASCICULE DE BREVET EUROPEEN

(11) **EP 0 994 656 B1**
(45) Date de publication et mention de la délivrance du brevet: **19.04.2006**
(21) Numéro de dépôt: 98939516.5
(22) Date de dépôt: 12.06.1998
(51) Int. Cl.: A23G 9/00, A23C 9/123, A23L 1/0528, A23L 1/308

(54) **dessert congelé avec bactéries lactiques et fibres fermentescibles**
gefrorenes Dessert, welches Milchsäurebakterien und fermentierbare Ballaststoffe enthält
frozen dessert comprising lactic acid bacteria and fermentable fibre

(30) Priorité: 05.07.1997 EP 97111382
(43) Date de publication de la demande: 26.04.2000
(73) Titulaire: SOCIETE DES PRODUITS NESTLE S.A., 1800 Vevey (CH)
(72) Inventeur: LESENS, Corinne, F-60000 Beauvais (FR); PFEIFER, Andrea, M., A., CH-1806 Saint-Legier (CH); ROCHAT, Florence, CH-1820 Montreux (CH)
(74) Mandataire: Archambault, Jean
(86) Numéro de dépôt international: PCT/EP1998/003721
(87) Numéro de publication internationale: WO 1999/002042

(56) Documents cités:
- EP-A- 0 307 523
- EP-A- 0 726 272
- WO-A-95/17103
- WO-A-97/29762
- US-A- 4 435 389
- H.W. MODLER ET AL.: "Using Ice Cream as a Mechanism to Incorporate Bifidobacteria and Fructooligosaccharides into the Human Diet" CULTURED DAIRY PRODUCTS JOURNAL, vol. 25, no. 3, 1990, pages 4-6, 8-9, XP002051107 CANADA
- H.D.GOFF ET H.W.MODLER: "Bifidobacteria, Fructooligosaccharides and Ice Cream" CANADIAN DAIRY, vol. 75, no. 3, 1996, page 10 XP002051108
- DATABASE CAB CAB INTERNATIONAL, WALLINGFORD, OXON, GB NAKAKUKI T: "Development of gentiooligosaccharide-containing syrups and their properties." XP002051109 & FOODS AND FOOD INGREDIENT JOURNAL OF JAPAN, 1996,
- PATENT ABSTRACTS OF JAPAN vol. 096, no. 002, 29 février 1996 & JP 07 278170 A (ENSUIKO SUGAR REFINING CO LTD), 24 octobre 1995
- PATENT ABSTRACTS OF JAPAN vol. 015, no. 243 (C-0842), 21 juin 1991 & JP 03 076561 A (UNIE KOROIDO KK), 2 avril 1991
- PATENT ABSTRACTS OF JAPAN vol. 097, no. 010, 31 octobre 1997 & JP 09 154535 A (SANWA KAGAKU KENKYUSHO CO LTD), 17 juin 1997
- DATABASE WPI Section Ch, Week 9519 Derwent Publications Ltd., London, GB; Class B04, AN 95-143788 XP002089001 & JP 07 067575 A (SUNTORY LTD) , 14 mars 1995
- DATABASE WPI Section Ch, Week 9303 Derwent Publications Ltd., London, GB; Class B03, AN 93-022694 XP002089002 & JP 04 349868 A (ASAHI CHEM IND CO LTD) , 4 décembre 1992
- DATABASE WPI Section Ch, Week 9115 Derwent Publications Ltd., London, GB; Class B04, AN 91-106851 XP002089003 & JP 03 049670 A (MITSUI TOATSU CHEM INC) , 4 mars 1991

## Description

La présente invention se rapporte aux effets bénéfiques sur la santé humaine des desserts congelés contenant des bactéries lactiques et des fibres alimentaires.

### État de la technique

Bien que les bactéries lactiques soient généralement connues pour avoir des effets bénéfiques sur la santé humaine, seulement certaines catégories de bactéries lactiques, appelées bactéries probiotiques, sont réellement capables d'adhérer aux cellules intestinales humaines, d'exclure des bactéries pathogènes sur des cellules intestinales humaines, et/ou d'agir sur le système immunitaire humain en lui permettant de réagir plus fortement à des agressions externes. Parmi les bactéries lactiques reconnues comme telles, on peut actuellement distinguer les souches *Lactobacillus plantarum* 299, *Lactobacillus rhamnosus* ATCC53103, *Lactobacillus acidophilus* CNCM I-1225, *Bifidobacterium breve* CNCM I-1226, *Bifidobacterium infantis* CNCM I-1227 et *Bifidobacterium longum* CNCM I-1228 (EP577904; EP577903; EP199535; US5591428; Gut, 35, 483-489, 1994; J. of Dairy Science, 78, 491-497, 1995; Applied Env. Microb., 59, 4121-4128, 1993), par exemple.

L'utilisation des propriétés bénéfiques des bactéries lactiques n'a pas échappé au domaine des desserts congelés. US5112626 (Pillsbury) propose en effet de préparer un yogourt fermenté par *Lactobacillus bulgaricus* et *Streptococcus thermophilus,* puis de le foisonner et le congeler. De même, Hekmat *et al.* proposent de préparer des crèmes glacées qui ont été fermentées par des bactéries lactiques connues pour être particulièrement bénéfiques pour la santé humaine (J. Dairy Science, 75, 1415-1422, 1992), par exemple.

Les fibres alimentaires prébiotiques sont généralement de nature protéique ou saccharidique se comportant comme des facteurs de croissance pour certaines bactéries lactiques. La littérature ayant trait à ces fibres est abondante, et l'on peut citer, à titre d'exemple, les brevets EP726272 (Hayashibara Seibutsu KK), US4435389 (Yakult Honsha KK), et les articles de T. Nakakuti (Foods and Food Ingredient J. of Japan, 167, 116-121, 1996) et de Playne *et al*. (Bulletin of the IDF 313, Group B42, Annual Session of September 95, Vienna).

L'utilisation simultanée de bactéries lactiques et de fibres alimentaires a aussi été proposée pour la préparation de desserts congelés.

Ainsi, M.W. Modler *et al*. rapportent qu'une crème glacée contenant des bifidobactéries et des fructooligosaccharides présente un intérêt remarquable pour la santé humaine (Cult. Dairy Prod. J.., 25, p. 4-9, 1990; Canadian Dairy, 75, p. 10, 1996). De même, EP307523 (Yakult Honsha KK) rapporte qu'un lait fermenté contenant des fibres prébiotiques peut être conditionné sous la forme d'une crème glacée, et être ainsi utilisée pour traiter certains désordres gastro-intestinaux.

Cependant, le fait de mettre en contact des fibres alimentaires et les bactéries lactiques présente des inconvénients non négligeables, ayant une incidence directe sur la santé humaine. Ces inconvénients sont d'ordre divers, et ont trait notamment à la destruction prématurée des fibres lors de la préparation et du stockage du dessert, et aux mauvaises conditions *in vivo* dans lesquelles l'activité biologique de ces fibres se développe, par exemple.

A ce jour, ces inconvénients, ni d'ailleurs aucun produit tel que défini dans la présente invention, n'ont été rapportés. La présente invention se destine ainsi à potentialiser l'effet bénéfique pour la santé humaine des desserts congelés contenant des bactéries lactiques et des fibres alimentaires.

### Résumé de l'invention

A cet effet, l'invention concerne un dessert congelé à base d'une crème glacée contenant des bactéries lactiques, ladite crème glacée étant enrobée sur tout ou partie de sa surface par, et/ou disposée dans, et/ou entre, un support ingestible, caractérisé en ce que le support est dépourvu substantiellement de bactéries lactiques, et qu'il comprend des fibres alimentaires fermentescibles favorisant spécifiquement la croissance dans le tractus intestinal des bactéries lactiques contenues dans la crème glacée.

L'invention a aussi pour objet l'utilisation d'une composition congelée selon la revendication 9.

### Description détaillée de l'invention

La crème glacée selon l'invention peut avoir toutes les compositions choisies par l'homme du métier, pour autant qu'elle soit foisonnée à raison de 20% à 200% en volume, par exemple.

De préférence cette crème comprend, après foisonnement et congélation, plus de 10⁶ cfu/g de bactéries lactiques, lesdites bactéries pouvant être choisies parmi les espèces *Lactococcus lactis,* notamment *L. lactis subsp. cremoris* et *L. lactis subsp. lactis biovar diacetylactis; Streptococcus thermophilus;* le groupes des bactéries acidophiles constitué de *Lactobacillus acidophilus, Lactobacillus crispatus, Lactobacillus amylovorous, Lactobacillus gallinarum, Lactobacillus gasseri* et *Lactobacillus johnsonii; Lactobacillus rhamnosus, Lactobacillus brevis; Lactobacillus fermentum; Lactobacillus plantarum; Lactobacillus helveticus; Lactobacillus casei* notamment *L. casei subsp. casei* et *L. casei subsp. rhamnosus; Lactobacillus delbruckii* notamment *L. delbruckii sbp. lactis,* et *L. delbruckii sbp. bulgaricus;* les bifidobacteries notamment *Bifidobacterium infantis, Bifidobacterium breve, Bifidobacterium longum;* et enfin *Leuconostoc mesenteroides* notamment *L. mesenteroides subsp cremoris,* par exemple (Bergey's Manual of Systematic Bacteriology, vol 2, 1986; Fujisawa *et al*., Int. Syst. Bact, 42, 487-491, 1992).

Les bactéries lactiques probiotiques présentent à cet effet un intérêt particulier dans le cadre de la présente invention. Ces bactéries sont en fait capables d'adhérer aux cellules intestinales humaines, d'exclure des bactéries pathogènes sur des cellules intestinales humaines, et/ou d'agir sur le système immunitaire humain en lui permettant de réagir plus fortement à des agressions externes (capacité d'immunomodulation), par exemple en augmentant les capacités de phagocytose des granulocytes issus du sang humain (J. of Dairy Science, 78, 491-497, 1995: capacité d'immunomodulation de la souche *La-1* qui a été déposée sous le traité de Budapest à la Collection Nationale de Culture de Microoganisme (CNCM), 25 rue du docteur Roux, 75724 Paris, où elle s'est vue attribuer le numéro de dépôt CNCM I-1225).

A titre d'exemple, on peut utiliser la souche probiotique *Lactobacillus acidophilus* CNCM I-1225 (voir EP577904, Société des Produits Nestlé). Cette souche a été récemment re-classifiée parmi les *Lactobacillus johnsonii*, suite à la nouvelle taxonomie, proposée par *Fujisawa et al*., qui fait maintenant autorité en matière de taxonomie des lactobacilles acidophiles (Int. J. Syst. Bact., 42, 487-791, 1992). D'autres bactéries probiotiques sont également disponibles, comme celles décrites dans EP199535 (Gorbach *et al*.), US5296221 (Mitsuoka *et al*.), US556785 (Institut Pasteur), ou US5591428 (Probi AB), par exemple.

De nombreuses méthodes sont à la disposition de l'homme du métier pour préparer une crème glacée foisonnée comprenant des bactéries lactiques vivantes. A cet effet, les procédés décrits dans DD154424, EP438201, SU1374465, FR2423163, NL9000101, US4293573, US4308287 et US5112626 peuvent être incorporés par préférence à la description de la présente invention, l'homme du métier étant à même de les adapter pour mettre en oeuvre la présente invention, par exemple.

Certains procédés de préparation sont cependant plus adaptés pour assurer un nombre important de bactéries lactiques vivantes dans la crème glacée foisonnée.

A cet effet, on peut incorporer un gaz neutre lors du foisonnement, comme du CO₂ ou de l'azote, seul ou en mélange, de manière à protéger les bactéries lactiques qui sont sensibles à l'oxygène, par exemple.

On peut aussi foisonner la crème à 130-200% en volume, puis y incorporer un lait fermenté par des bactéries lactiques pour atteindre un foisonnement final de l'ordre de 80-150% en volume, par exemple.

Afin d'assurer un nombre important de bactéries lactiques vivantes dans la crème glacée foisonnée, la température en sortie du batteur est aussi considéré comme un paramètre important. Par exemple, une crème foisonnée à environ 95% et refroidie à environ -3°C en sortie de batteur contient significativement plus de bactéries vivantes (10⁷ cfu/g) qu'une crème foisonnée à environ 95% et refroidie à environ -6°C qui en contient environ 2 à 10 fois moins (5 à 1 x 10⁶ cfu/g). Cette différence est maintenue après durcissement de la crème glacée et après 1, 3, et 6 mois de stockage à -30°C.

Un autre intérêt incident à l'addition d'un lait fermenté au cours de la fabrication d'une crème glacée foisonnée est de développer une texture très onctueuse, très crémeuse, même si le taux de matière grasse est inférieur ou égal à 8%, et de développer un arôme d'origine lactique ayant une note beurrée. Cette texture crémeuse est maintenue, pendant plusieurs semaines, lors des tests de vieillissement accéléré (cycles successifs de températures étalés sur 24 h respectants des paliers à -10°C, -20°C et -30°C, puis à -30°C, -20°C et -10°C). La crème glacée foisonnée, contenant un lait fermenté, a aussi une très bonne résistance au test de fusion (mesure du poids de crème glacée fondue en fonction du temps, quand la crème glacée est maintenue dans une enceinte à +20°C). Par exemple, après plus de deux heures de test de fusion, seulement 40% à 50% de la crème glacée est sous forme liquide, le reste étant maintenue sous la forme d'une mousse. De plus, la taille des cristaux de glace, influençant directement le caractère « lisse » de la crème glacée, varie aussi très faiblement (environ 1 à 10 µm pour le diamètre moyen des cristaux) entre le début et la fin du vieillissement accéléré. L'intérêt de cette bonne résistance au test de fusion et, du maintien de la texture crémeuse au cours du stockage et de la faible variation de la taille des cristaux de glace au cours du vieillissement accéléré est, par exemple, de pouvoir remplir un support ingestible tel une gaufrette en forme de cône, et de former une "flamme" de l'ordre de 50 mm de hauteur, puis de pouvoir conserver cette forme en "flamme" au cours du stockage.

Dans une alternative de la présente invention, on peut choisir d'ajouter à la crème des bactéries lactiques encapsulées, sèches ou non, par exemple selon l'une des techniques décrites ci-après.

Le dessert congelé selon la présente invention comprend par ailleurs un support, avec lequel on enrobe, ou dans lequel ou entre lequel on dispose, la crème glacée selon l'invention. Ce support est dépourvu substantiellement de bactéries lactiques, ce qui signifie que l'on n'introduit pas sciemment des bactéries lactiques dans sa composition. Ce support contient en outre des fibres qui ne sont pas ou peu digérés dans l'estomac et le tractus intestinal, mais qui peuvent néanmoins être spécifiquement fermentées par les bactéries lactiques présents dans la crème glacée, permettant ainsi de restaurer ou de promouvoir une flore intestinale forte en bactéries lactiques bénéfiques.

Ces fibres peuvent être de nature protéique ou saccharidique choisies, par exemple, parmi les pectines végétales, les chito-, fructo-, gentio-, galacto-, isomalto-, manno- ou xylo-oligosaccharides, ou les oligosaccharide de soja, de *Polymnia sonchifolia*, d'artichaud, d'oignon ou d'asperge, ou les amidons résistants, ou les produits riches en β-glucanes tel qu'un concentré d'avoine, par exemple (Playne *et al*.; Fukai et al., Soil Sci. Plant Nutr., 39, 567-571, 1993).

Les pectines préférées sont des polymères d'acide α-1,4-D-galacturonique ayant un poids moléculaire de l'ordre de 10 à 400 kDa, que l'on peut purifier des carottes ou des tomates, par exemple (JP60164432). Les galacto-oligosaccharides préférés comprennent une partie saccharidique constituée de 2 à 5 unités répétitives de la structure [-α-D-Glu-(1→4)-β-D-Gal-(1→6)-] (Yakult Honsa Co., Japon). Les fructo-oligosaccharides préférés sont des inulin-oligofructose extraits de la chicorée pouvant comprendre, par exemple, 1-9 unités répétitives de la structure [-β-D-Fru-(1→2)-β-D-Fru-(1→2)-] (WO94/12541; Raffinerie Tirlemontoise S.A., Belgique), ou des oligosaccharides synthétisés à partir d'unités saccharose pouvant comprendre, par exemple, une partie saccharidique constituée de 2 à 9 unités répétitives de la structure [-α-D-Glu-(1→2)-β-D-Fru-(1→2)-] (Meiji Seika Kasiha Co., Japon). Les malto-oligosaccharides préférés comprennent une partie saccharidique constituée de 2 à 7 unités répétitives de la structure [-α-D-Gal-(1→4)-] (Nihon Shokuhin Kako Co., Japon). Les isomaltose préférés comprennent une partie saccharidique constituée de 2 à 6 unités répétitives de la structure [-α-D-Glu-(1→6)-] (Showa Sangyo Co., Japon). Les gentio-oligosaccharides préférés comprennent une partie saccharidique constituée de 2 à 5 unités répétitives de la structure [-β-D-Glu-(1→6)-] (Nihon Shokuhin Kako Co., Japon). Enfin, les xylo-oligosaccharides préférés comprennent une partie saccharidique constituée de 2 à 9 unités répétitives de la structure [-β-xyl-(1→4)-] (Suntory Co., Japon), par exemple.

La quantité de fibres dans le dessert selon l'invention dépend de la capacité de celles-ci à promouvoir le développement des bactéries lactiques. En règle générale, le support peut contenir de 0,1 à 20% de telles fibres (en poids sur la matière sèche). Autrement dit, le dessert peut comprendre au moins 10³ cfu de bactéries lactiques par g de fibres, de préférence 10⁴ à 10⁷ cfu/g de fibres, par exemple.

Par ailleurs, le dessert peut être conçu de sorte à pouvoir fournir potentiellement jusqu'à un maximum 10 g de fibres par dessert, des quantités supérieures en fibres induisant en effet une sensation de lourdeur désagréable dans l'estomac (Bouhnik *et al*., Cah. Nutr. Diet., 6, 418-422, 1991; Ito *et al*., Microb. Ecol. Health Dis., 3, 285-292, 1990).

L'originalité de la présente invention réside particulièrement dans le fait que les bactéries lactiques ne sont pas en contact substantiel avec les fibres, ce qui évite une fermentation intempestive des fibres lors de la préparation du dessert, en particulier lorsque l'on fermente la crème au cours du procédé de fabrication du dessert selon l'invention (voir l'exemple 2).

Par ailleurs, il a été observé que plus la diète contient un concentrât solide de fibres, plus le transit intestinale de ces fibres est retardé, influençant d'autant positivement le développement des bactéries lactiques dans l'intestin. Au cours d'une étude sur des volontaires humains, il a ainsi été mis en évidence que les troubles digestifs, liés probablement à une fermentation bactérienne trop forte et limités dans le temps, sont plus marqués lorsque des sujets sont nourris avec des fructo-oligosaccharides (FOS) et des bactéries lactiques conditionnés en une solution liquide buvable, et cela en comparaison des résultats obtenus avec une diète contenant la même quantité de FOS conditionnés en présence d'aliments solides et de bactéries lactiques.

En résumé, plus l'on concentre les fibres dans le dessert selon l'invention, par exemple en les plaçant dans un support solide distinct de la crème glacée (enrobage, gaufrette, etc.), plus on favorise une fermentation bactérienne dans l'intestin qui est prolongée et suffisante pour satisfaire les besoins du corps humain.

Un autre problème incident résolu par la présente invention réside dans le fait que certaines fibres peuvent être facilement dégradées par le pH acide développé par les bactéries lactiques. Cette dégradation peut être observée lorsque l'on désire fermenter une crème contenant ces fibres, ou bien lorsque l'on soumet une crème glacée contenant des fibres et des bactéries lactiques à un traitement de vieillissement accéléré. Encore une fois, le fait de séparer les fibres de la crème glacée fermentée permet donc de bien potentialiser l'effet bénéfique des bactéries lactiques sur la santé humaine.

Dans une première forme de réalisation de l'invention, le support ingestible peut être une composition servant traditionnellement à enrober une crème glacée. L'enrobage peut être réalisée classiquement par pulvérisation, trempage ou moulage, par exemple. A titre d'indications, les techniques et les compositions d'enrobage décrites dans US4985263, WO95/21536 et FR2680635 sont incorporées par référence à la description de la présente invention.

De préférence, au moins une partie de l'enrobage a été fermentée par des bactérie lactiques, puis pasteurisé de sorte à tuer toutes les bactéries lactiques et à uniquement conserver les propriétés de textures fournies par celles-ci. Dans ce cadre, on peut utiliser des bactéries lactiques productrices de polysaccharides texturants, notamment celles décrites dans EP95201669.9 et EP96201535.0. Sans vouloir être limité par l'aspect scientifique, il semble en effet que les polysaccharides texturants sont impliqués dans la capacité de l'enrobage à correctement adhérer à la crème glacée, et à être, à la fois, souple et croquant.

L'enrobage peut ainsi comprendre 1% à 70% d'un lait fermenté par les bactéries lactiques (puis inactivé), 0,5% à 5% de protéines animales ou végétales, et un taux de matières grasse de 2% à 20%, lesdites matières grasses pouvant être d'origine lactique, lesdites protéines pouvant être des protéines du jaune d'oeuf ou du sérum du lait, par exemple. On peut noter qu'il n'est pas nécessaire d'ajouter des composés riches en matière grasse, végétale ou non, de façon à obtenir des taux de matière grasse proches de 40%, car la texture en bouche de l'enrobage congelé selon l'invention rappelle en effet celle d'un enrobage congelé traditionnel ayant de 40% à 50% en matière grasse végétale, par exemple.

Un dessert répondant parfaitement aux conditions énoncées ci-dessus peut comprendre une partie crème glacée foisonnée comprenant plus de 10⁶ cfu/g de bactéries lactiques, et une partie enrobage qui a été fermenté par des bactéries lactiques jusqu'à un taux de 5x10⁶ cfu/g, puis inactivé à la chaleur, et qui comprend 0, 1 % à 10% d'un fructo-oligosaccharide, 1% à 60% d'un lait, 0,5% à 5% de protéines animales ou végétales, un taux de saccharose de 15% à 30% et un taux de matière grasse lactique de 2% à 20%, par exemple.

Le dessert peut ainsi se présenter sous la forme d'une sucette glacée enrobée, c'est à dire qu'il comporte un bâtonnet de préhension en son centre. La partie crème glacée foisonnée de la sucette sera formée selon la technique de moulage ou d'extrusion, par exemple. La sucette peut avoir un profil polygonal, par exemple rectangulaire, triangulaire, carré, en étoile, etc., ou un profil elliptique ou circulaire, par exemple.

Dans une deuxième forme de réalisation de l'invention, le support ingestible peut être un article de boulangerie ou de pâtisserie, par exemple une gaufrette, un biscuit et/ou une génoise ayant la forme d'un cornet ou d'une feuille dans lequel, ou sur lequel, ou entre lequel, on peut disposer la crème glacée. Le dessert congelé peut donc avoir la forme d'un cône glacé, d'un sandwich glacé, d'une cigarette fourrée, d'un chausson glacé, d'une crêpe fourrée ou d'un gâteau glacé, par exemple.

L'homme du métier dispose de nombreuses compositions de pâte destinées à être combinées à une crème glacées dans le cadre de la préparation d'un dessert congelé. A titre d'indications, les compositions de gaufrettes ou biscuits, et/ou les techniques pour les fabriquer, décrites dans FR1454750, US3793938, US4624855, WO95/32630, US4761293 et GB2167934 sont parfaitement adaptables par l'homme du métier, et sont donc incorporées par référence dans la description de la présente invention.

Dans les cas préférés, l'article de boulangerie ou de pâtisserie est revêtu, sur la face en contact avec la crème glacée, d'une fine couche de couverture grasse, par exemple de chocolat, jouant le rôle de barrière contre l'influence de l'humidité et des bactéries lactiques.

L'invention a aussi pour objet l'utilisation combinée de bactéries lactiques et de fibres prébiotiques, pour la préparation d'une composition congelée dans laquelle les bactéries lactiques et les fibres ne sont pas substantiellement en contact, pour le traitement et/ou la prévention des désordres gastro-intestinaux, pour renforcer le système immunitaire humain, ou pour augmenter l'absorption des minéraux.

Cette composition peut être l'un des desserts congelés décrits ci-dessus, ou même un mélange de fibres prébiotiques et de bactéries lactiques encapsulées, sèches ou non, par exemple. On a d'ailleurs trouvé que la micro-encapsulation des bactéries présente des avantages technologiques et thérapeutiques indéniables. Premièrement, la micro-encapsulation augmente significativement la survie des bactéries lactiques, et donc le nombre de bactéries lactiques vivantes qui arrivent dans l'intestin. Encore plus important, les bactéries lactiques sont graduellement relâchées dans l'intestin, ce qui permet une action prolongée des bactéries lactiques.

De préférence, pour encapsuler les bactéries lactiques, on sèche les bactéries lactiques par lyophilisation ou par pulvérisation (EP96201922.0), et on les incorpore dans un gel formé, par exemple, par un acide gras solidifié, un alginate de sodium, de l'hydroxypropylmethylcellulose polymérisé ou du polyvinypyrrolidone polymérisé. A cet effet, l'enseignement dispensé dans FR2443247 (Société des Produits Nestlé) est incorporé par référence à la description de la présente invention.

Les désordres gastro-intestinaux peuvent être de natures diverses, généralement liés à une mauvaise balance de la flore intestinale, pouvant ainsi conduire à des problèmes de constipations ou de diarrhées. On sait aussi que le système immunitaire humain est particulièrement sensibles à des composés produits par les bactéries lactiques. La souche *Lactobacillus johnsonii* CNCM I-1225 est à ce titre un candidat idéal pour remplir les besoins de la présente invention. Par ailleurs, on sait également maintenant que les bactéries lactiques peuvent être directement impliquées dans l'absorption facilitée des minéraux, tel que le calcium, le magnésium, le fer et/ou le zinc, par exemple (voir EP97111380.8, Société des Produits Nestlé).

La présente invention est décrite plus en détail par les exemples présentés ci-après. Il va de soi, toutefois, que ces exemples sont données à titre d'illustration de l'objet de l'invention dont ils ne constituent en aucune manière une limitation. Les pourcentages sont donnés en poids sauf indication contraire. Dans ces exemples, les souches *Lactobacillus johnsonii LA-1, Bifidobacterium longum B116* et *Streptococcus thermophilus Sfi21* ont été respectivement déposées, uniquement à titre d'exemple, le 30 juin 1992 (*La-1* et *B116*) et le 18 mai 1994 (*Sfi21*), à la Collection Nationale de Cultures de Microorganismes (CNCM), 25 rue du docteur Roux, 75724 Paris, France, où elles ont reçues respectivement les numéros de dépôt CNCM I-1225, CMCM I-1228 et CNCM I-1424.

### Exemple 1 Crème glacée foisonnée comprenant une addition de lait fermenté

On prépare une base concentrée pour crème glacée, en mélangeant à 60-65°C pendant 20 min, environ 11 % de graisses lactiques, 8,8% de solides de lait (non gras), 25% saccharose, 5% de sirop de glucose et 0,6% d'Emulstab® SE30. On homogénéise la base à 72-75°C et à 210 bars (2 étages à 210/50 bars), on la pasteurise à 85°C pendant 22 sec (pasteurisateur APV, France, Evreux, 400 1/h), on la refroidit à 4°C, et on y ajoute 40% d'un lait acidifié par les souches *Lactobacillus johnsonii La-1* (10⁷ cfu/ml) et *Bifidobacterium longum B116* (10⁷ cfu/ml). La composition de la crème ainsi préparée est exposée dans le tableau 1 ci-dessous.

**Tableau 1**

| Ingrédients | Composition (kg) | Graisses (%) | Matières sèches non grasses (%) | Saccharose (%) | Extrait sec (%) |
|---|---|---|---|---|---|
| Crème (35%) | 31,43 | 11,00 | 1,57 | | 12,57 |
| Poudre de lait écrémé | 7,60 | | 7,30 | | 7,30 |
| Saccharose | 36,77 | | | 25,00 | 25,00 |
| Sirop de glucose | 5,27 | | | | 5,00 |
| Emulstab® SE30 | 0,67 | | | | 0,63 |
| Eau | 18,26 | | | | |
| Total: base crème | 100,00 | 11,00 | 8,87 | 25,00 | 50,50 |
| Base crème (60%) | 60,00 | 6,60 | 5,32 | 15,00 | 30,30 |
| Lait acidifié (40%) | 40,00 | 1,40 | 4,68 | - | 6,08 |
| Total: Base crème + lait acidifié | 100,00 | 8,00 | 10,00 | 15,00 | 36,38 |

Après maturation de la crème pendant 12h à 4°C, on la glace à un degré de foisonnement de 95% en volume (glaceur Crepaco, France, Evreux; 1601. de produit/h), puis on enrobe tout ou partie de la crème foisonnée avec les différentes compositions d'enrobage décrites ci-après.

Pour préparer un bâtonnet moulé, on utilise la technique classique de remplissage "shell and core". A cet effet, l'enrobage dont la composition est donnée dans le tableau 2 ci-après est dosé dans un moule, ledit moule étant disposé dans un bain à eau glycolée à -35°C. Après 30 s, l'enrobage non congelé est aspiré. Seul l'enrobage congelé reste dans le moule, pour former une coque. La crème glacée foisonnée à 95%, sortie du glaceur à -3°C est alors dosée dans cette coque. Après 30 min. d'attente, le bâtonnet est démoulé. Il est ensuite pulvérisé avec de l'eau de source, emballé, placé pendant 3 heures en chambre de durcissement à -40°C, puis stocké en chambre à -30°C.

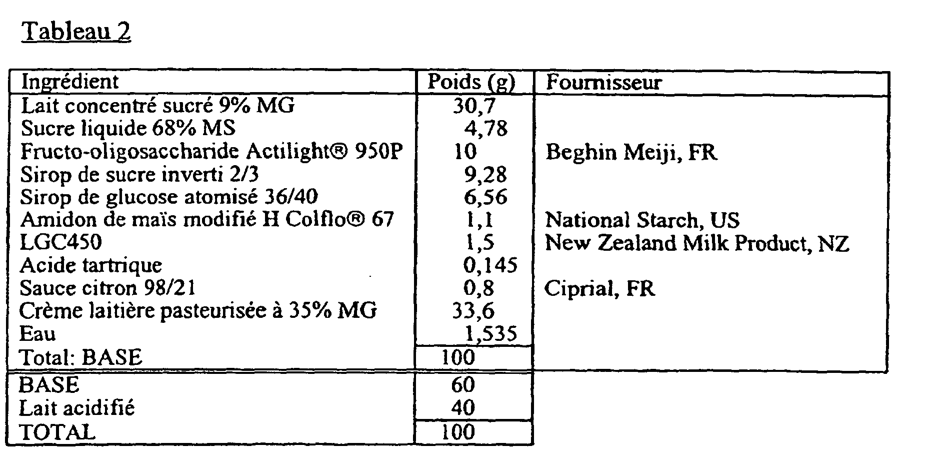

Pour préparer un bâtonnet extrudé, on extrude la crème glacée foisonnée à 95%, sortie à -5°C/-6°C du glaceur, avec un extrudeur ayant une tête d'extrusion ayant une forme souhaitée. Le bâtonnet extrudé est ensuite congelé en tunnel de congélation à -45°C. Le bâtonnet est alors enrobé par trempage dans la composition présentée dans le tableau 3 ci-après. Il est ensuite pulvérisé avec de l'eau de source, emballé, placé pendant 3 heures en chambre de durcissement à environ -40°C, puis stocké en chambre à -30°C.

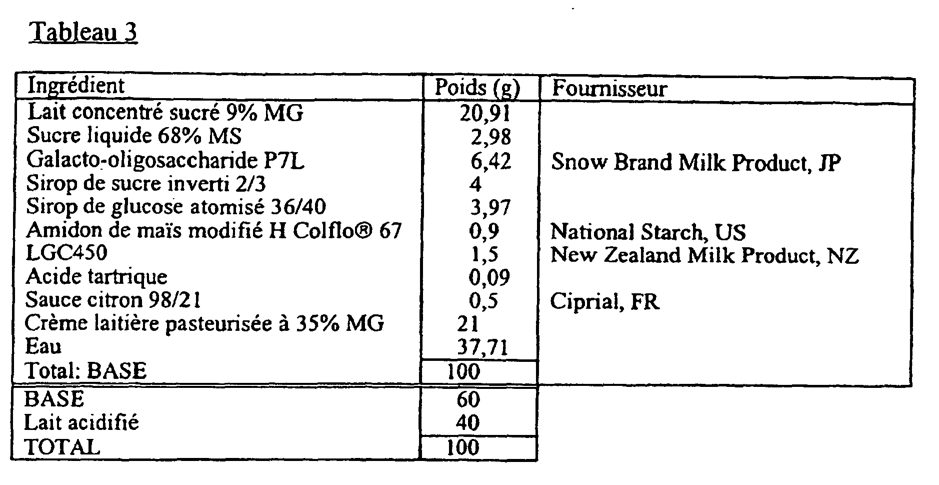

Pour préparer un pot de crème glacée, on extrude la crème glacée foisonnée à 95%, sortie à -5°C/-6°C du glaceur, avec un extrudeur ayant une tête d'extrusion à la forme souhaitée, dans des pots 12 cl. Par dosage, le décor, dont la composition est décrite dans le tableau 4 ci-après, est déposé à la surface de la crème glacée foisonnée. Le pot est placé, pendant 3 heures, en chambre de durcissement à environ -40°C, puis il est stocké en chambre à -30°C.

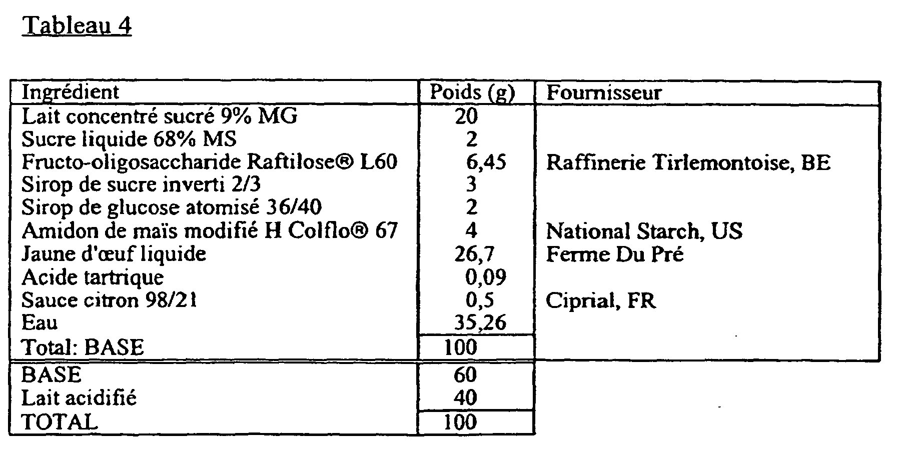

On soumet les bâtonnets glacés et les pots, à des cycles successifs de températures étalés sur 24h, respectants des paliers à -10°C, -20°C et -30°C, puis à -30°C, -20°C, et -10°C. La crème glacée est alors soumise à un vieillissement accéléré. On analyse ensuite la survie des bactéries au cours du temps, ainsi que la stabilité des fibres prébiotiques. Dans tous les desserts, on observe uniquement un faible déclin, de l'ordre de 20%, du nombre de bactéries lactiques après 3 mois de vieillissement accéléré. Les fibres prébiotiques sont également parfaitement stables dans ces conditions.

Enfin, la capacité des bâtonnets enrobés à promouvoir le développement des bactéries lactiques *La-1* et *B116* dans l'intestin est également mis en évidence, en déterminant le nombre de *La-1* et *B116* résidant dans les fèces après plusieurs jours suivant une consommation régulière d'environ 200 ml, soit environ 100g, de crèmes glacées par jour, et cela au regard d'une diète dépourvue en fibres.

### Exemple 2 Crème glacée foisonnée et fermentée

On prépare une crème comprenant 10,8% de graisses lactiques, 13,5% de solides de lait (non gras), 0,3% d'Emulstab® SE30, et 0,3% d'Emulstab® mousse (Grindsted, DK), on la pasteurise à 105°C pendant 20 s, on l'homogénéise à 75°C et 300 bars, on la refroidit à 38°C, et on l'inocule avec des précultures en milieu MRS, prises en phase exponentielle de croissance, à raison de 5% en poids d'une préculture de *La-1*, et 0,5% en poids d'une préculture de la souche *Streptococcus thermophilus Sfi21*. On fermente ensuite la crème pendant 10h à 38°C jusqu'à un pH d'environ 4,5. A la fin de la fermentation, on y ajoute du saccharose et un sirop de glucose. La composition de la crème est présentée dans le tableau 5 ci-après.

**Tableau 5**

| Ingrédients | Composition (kg) | Graisses (%) | Matières sèches non grasses (%) | Saccharose (%) | Extrait sec (%) |
|---|---|---|---|---|---|
| Crème (35%) | 30,83 | 10,79 | 1,54 | | 12,33 |
| Poudre de lait écrémé | 12,45 | | 11,95 | | 11,95 |
| Emulstab® SE30 | 0,41 | | | | 0,37 |
| Emulstab® mousse | 0,41 | | | | 0,36 |
| Eau | 55,91 | | | | |
| Total: base crème | 100,00 | 10,79 | 13,49 | - | 25,01 |
| Base crème | 74,14 | 8,00 | 10,00 | - | 18,54 |
| Saccharose | 22,06 | | | 15,00 | 15,00 |
| Sirop de glucose | 3,80 | | | | 3,00 |
| Crème glacée fermentée | 100,00 | 8,00 | 10,00 | 15,00 | 36,54 |

Le mélange est ensuite brassé, refroidi à 4°C, conservé à 4°C, glacée à un degré de foisonnement de 95% en volume (glaceur Crepaco, France; 160 1. de produit/h), puis enrobé selon les méthodes décrites à l'exemple 1 avec les différentes compositions d'enrobage décrites dans cet exemple, ou avec la composition d'enrobage "shell and core" aux oeufs donnée dans le tableau 6 ci-après.

**Tableau 6**

| Ingrédient | Poids (g) | Fournisseur |
|---|---|---|
| Lait concentré sucré 9% MG | 20 | |
| Sucre liquide 68% MS | 3 | |
| Oligosaccharides de soja | 6,45 | The Calpis Food Ind., JP |
| Sirop de sucre inverti 2/3 | 4 | |
| Sirop de glucose atomisé 36/40 | 4,2 | |
| Amidon de maïs modifié H Colflo® 67 | 0,8 | National Starch, US |
| Jaune d'oeuf liquide | 25,7 | Ferme Du Pré |
| Acide tartrique | 0,09 | |
| Sauce citron 98/21 | 0,5 | Ciprial, FR |
| Eau | 35,26 | |
| Total: enrobage | 100 | |

Après durcissement, le nombre de *La-I* vivantes dans les crèmes glacées foisonnées est de l'ordre de 7 x 10⁶ cfu/g, et après 3 mois de conservation à -30°C de l'ordre de 6 x 10⁶ cfu/g.

Les bâtonnets glacés et les pots soumis à un vieillissement accéléré comme décrit à l'exemple 1, survivent particulièrement bien, puisque moins de 50% des bactéries lactiques meurent. Les fibres prébiotiques restent également stables dans ces conditions.

La capacité de l'enrobage à adhérer à la crème glacée, et à être souple et croquant est également évaluée par un panel de dégustateurs. Les résultats montrent que les bâtonnets enrobés présentent après 1 mois de vieillissement accéléré aucune altération concernant l'adhérence, la souplesse et le croquant.

Enfin, la capacité des bâtonnets enrobés à promouvoir le développement des bactéries lactiques dans l'intestin est également mis en évidence, en déterminant le nombre de bactéries lactiques résidant dans les fèces après plusieurs jours suivant une consommation régulière d'environ 200 ml de crèmes glacées par jour, et cela au regard d'une diète dépourvue en fibres.

### Exemple 3 Encapsulation de bactéries lactiques

Dans une cuve de 100 1, on prépare 80 1 de milieu de culture présentant la composition suivante: 0,25% d'extrait de levure, 1,00% de trypticase, 0,50% de phytone, 1,5% de glucose, 0,05% de L-cysteine HCl, 0,25% de K2HPO4, 0,025% de ZnSO4, des traces de FeC13, et le reste d'eau.

On inocule avec 1 1 d'une culture de 20 h de *Bifidobacterium longum* CNCM-1228. On incube le milieu pendant 12 h à 30°C. On centrifuge le bouillon de culture et l'on récolte 240 g de cellules. On les dilue dans 250 ml de lait écrémé additionné de 7% de lactose. On congèle le mélange à l'azote liquide. On lyophilise à 40°C pendant une nuit. On prépare une dispersion à 5% de la poudre obtenue dans de la graisse végétale hydrogénée présentant un point de fusion de 42°C et liquéfiée à 45°C. On injecte la dispersion à 45°C sous une pression de 4 bars, en même temps que de l'azote liquide, à raison de 1 partie de dispersion pour 5 parties d'azote, au sommet d'un cylindre vertical de 1,5m de diamètre et 10 m de hauteur. Au fond du cylindre est placé un récipient contenant de l'azote liquide dans lequel on recueille des microbilles renfermant des bifidobactéries dont le diamètre oscille entre 0,1 mm et 0,5 mm. On met ensuite les microbilles en lit fluidifié et l'on pulvérise sur le lit une solution alcoolique à 8% de zéine, en quantité telle que la couche de zéine formée autour des microbilles représente 5% de leur poids.

Les microbilles sont ensuite incorporées à la même crème glacée décrite à l'exemple 1 (voir le tableau 1 ci-dessus), à raison d'au moins 10⁵ cfu/g, à la différence près que l'on remplace les 40% de lait acidifié par simplement du lait pasteurisé. Puis on enrobe la crème glacée selon l'une des techniques décrites à l'exemple 1.

### Exemple 4 Cône glacé

On prépare une pâte de gaufrette, contenant 10% de fructo-oligosaccharide Raftilose® L30 (Raffinerie Tirlemontoise S.A., BE), selon la recette reprise dans le tableau 7. Après cuisson, la gaufrette est mise classiquement en forme de cône. Après refroidissement, l'intérieur des cônes est recouvert par pulvérisation d'un film gras, puis les cônes sont remplies avec la crème glacée foisonnée décrite à l'exemple 1. Pour cela, la crème glacée de remplissage est préparée à - 3°C, afin d'avoir un nombre important de bactéries *La-1* vivantes. La flamme de 50 mm est constituée de la crème glacée foisonnée décrite à l'exemple 1, mais préparée à -5°C, pour faciliter le formage de la flamme. Pour un cône en gaufrette de 11,5 g, on utilise ainsi 90 ml de crème glacée foisonnée (environ 45 g), 40 ml de décor en forme de flamme (environ 20g) sont déposés et 5 g de chocolat (pulvérisation et décor). Dans cet exemple, 1,1 g de fibres sont apportées par cornet glacé.

**Tableau 7**

| Ingrédient | Poids (g) | Fournisseur |
|---|---|---|
| Farine de blé 55 ordinaire | 52 | |
| Amidon | 0,2 | |
| Fructo-oligosaccharide Raftilose® L30 | 10 | Raffinerie Tirlemontoise S.A., BE |
| Sucre | 27,8 | |
| Matière grasse | 8 | |
| Émulsifiant | 1,5 | |
| Sel | 0,5 | |
| Total: recette gaufrette | 100 | |

### Exemple 5 Cigarette russe glacée

On prépare des tubes en pâte de gaufrette fourrés par de la crème glacée pour former les cigarettes russes. La pâte de gaufrette reprend la recette décrite au tableau 7 ci-dessus, à la différence près qu'elle contient, à la place de Raftilose® L30, 10% de galacto-oligosaccharide P7L (produit non-dégradé lors de la cuisson: Snow Brand, japon). A titre d'indication, avant l'enroulement de la bande de gaufrette fraîchement cuite, on dispose par pulvérisation sur la face interne une fine couche de chocolat, on enroule la bande de gaufrette pour former un tube continu, on fourre le tube de pâte avec la crème glacée décrites à l'exemple 1, on tronçonne le tube de gaufrette de façon continue en tubes de pâte ayant une longueur voulue, et on enrobe les extrémités des tubes de pâtes avec la composition d'enrobage présentée au tableau 3 ci-dessus, et on les réfrigère à -20°C.

### Exemple 6 Sandwich glacé

On prépare une pâte de biscuiterie, contenant 10% d'isomalto-oligosaccharide Panorich® selon la recette reprise dans le tableau 8. La pâte de biscuiterie est mise en forme dans une machine rotative. Après cuisson et refroidissement, les biscuits sont garnis de la crème glacée foisonnée décrite à l'exemple 1. Pour deux biscuits de 10,5 g chacun, 100 ml de crème glacée foisonnée (environ 50 g) sont déposés. Pour cet exemple, 2,1 g de fibres sont apportés par sandwich.

**Tableau 8**

| Ingrédient | Poids (g) | Fournisseur |
|---|---|---|
| Farine | 62 | |
| Sucre | 15 | |
| Isomalto-oligosaccharide Panorich® | 10 | Nihon Shokuhin Kako Co., Japon |
| Cacao poudre | 3,5 | |
| Matière grasse | 8 | |
| Emulsifiant | 0,4 | |
| Extrait de malt | 0,5 | |
| Poudre levante | 0,4 | |
| Sel | 0,1 | |
| Arôme vanille | 0,1 | |
| Total: recette biscuit | 100 | |

### Exemple 7 Roulé glacé

On prépare un roulé glacé, c'est à dire une feuille de biscuit revêtue d'une couche de crème glacée, qui est roulée sur elle-même puis réfrigérée. Le biscuit reprend la composition décrite au tableau 8 ci-dessus à la différence près qu'il contient environ 10% en poids de gentio-oligosaccharide Gentose® 80P (Nihon Shokuhin Kako Co., Japon; produit non-dégradé lors de la cuisson). La crème glacée est celle décrite à l'exemple 2.

## Revendications

1. Dessert congelé composite constitué:
- d'une crème glacée contenant des bactéries lactiques probiotiques et
- d'un support ingestible dépourvu substantiellement de bactéries lactiques,
dans lequel le support est un enrobage ou un article de boulangerie ou de pâtisserie et comprend des fibres fermentescibles favorisant spécifiquement la croissance dans le tractus intestinal des bactéries lactiques contenues dans la crème glacée.

2. Dessert congelé selon la revendication 1, **caractérisé en ce que** les fibres sont choisies parmi les pectines végétales; ou les chito-, fructo-, gentio-, galacto-, isomalto-, manno- ou xylo-oligosaccharides; ou les oligosaccharides de soja, de *Polymnia sonchifolia*, d'artichaud, d'oignon ou d'asperge; ou les amidons résistants; ou les produits riches en β-glucanes.

3. Dessert congelé selon la revendication 1, **caractérisé en ce que** la crème glacée comprend plus de 10⁶ cfu/g de bactéries lactiques.

4. Dessert congelé selon la revendication 1, **caractérisé en ce que** la crème glacée comprend des bactéries lactiques encapsulées.

5. Dessert congelé selon la revendication 1, **caractérisé en ce que** le support ingestible est un un enrobage du type crème glacée non-foisonnée, ou un article de boulangerie ou de pâtisserie.

6. Dessert congelé selon la revendication 1, **caractérisé en ce que** les bactéries lactiques sont choisies parmi les bactéries acidophiles, les bifidobacteries, et les espèces *Lactococcus lactis, Lactobacillus rhamnosus, Lactobacillus fermentum, Lactobacillus brevis, Lactobacillus plantarum, Lactobacillus casei, Lactobacillus delbruckii sbp. bulgaricus, Lactobacillus delbruckii sbp. lactis, Streptococcus thermophilus* et *Leuconostoc mesenteroides.*

7. Dessert congelé selon la revendication 6, **caractérisé en ce que** les bactéries lactiques sont des bactéries capables d'adhérer aux cellules intestinales humaines, d'exclure des bactéries pathogènes sur des cellules intestinales humaines, et/ou d'agir sur le système immunitaire humain en lui permettant de réagir plus fortement à des agressions externes.

8. Procédé de fabrication d'un dessert selon l'une des revendications 1 à 7, dans lequel on prépare séparément une composition contenant les bactéries lactiques et un support contenant les fibres et on congèle le dessert de sorte que les dites bactéries ne sont pas substantiellement en contact avec les dites fibres,

9. Utilisation d'une composition congelée composite selon l'une des revendications 1 à 7, pour l'obtention d'un produit diététique destiné au traitement et/ou la prévention des désordres gastro-intestinaux, pour renforcer le système immunitaire humain, ou pour augmenter l'absorption des minéraux.

## Patentansprüche

1. Gefrorenes Dessert, bestehend aus:
- einer gefrorenen Creme, welche probiotische Milchsäurebakterien enthält, und
- einem im Wesentlichen von Milchsäurebakterien freien, essbaren Träger,
wobei der Träger eine Umhüllung oder ein Backwaren-Artikel oder ein Konditorei-Artikel ist, und fermentierbare Fasern umfasst, welche spezifisch das Wachstum der in gefrorener Creme bekannten Milchsäurebakterien im Intestinal-Trakt fördern.

2. Gefrorenes Dessert gemäß Anspruch 1, **dadurch gekennzeichnet, dass** die Fasern unter den pflanzlichen Pektinen; oder den Chito-, Frukto-, Gentio-, Galakto-, Isomalto-, Manno-, oder Xylo-Oligosachariden; oder den Oligosachariden von Soja, von Polymnia *sonchifolia,* von Artischocke, von Zwiebel oder von Spargel; oder den resistenten Stärken; oder den an β-Glucanen reichen Produkten ausgewählt sind.

3. Gefrorenes Dessert gemäß Anspruch 1, **dadurch gekennzeichnet, dass** die gefrorene Creme mehr als 10⁶ cfu/g Milchsäurebakterien umfasst.

4. Gefrorenes Dessert gemäß Anspruch 1, **dadurch gekennzeichnet, dass** die gefrorene Creme eingekapselte Milchsäurebakterien umfasst.

5. Gefrorenes Dessert gemäß Anspruch 1, **dadurch gekennzeichnet, dass** der essbare Träger eine Umhüllung vom Typ nicht-quellender gefrorener Creme oder ein Bäckerei-Artikel oder ein Konditorei-Artikel ist.

6. Gefrorenes Dessert gemäß Anspruch 1, **dadurch gekennzeichnet, dass** die Milchsäurebakterien ausgewählt sind unter: den acidophilen Bakterien, den Bifido-Bakterien und den Spezies *Lactococcus lactis,* Lactobacillus *rhamnosus, Lactobacillus fermentum, Lactobacillus brevis, Lactobacillus* plantarum, Lactobacillus casei, Lactobacillus delbruckii sbp. *bulgaricus, Lactobacillus delbruckii sbp. lactis, Streptococcus* thermophilus und *Leuconostoc mesenteroides.*

7. Gefrorenes Dessert gemäß Anspruch 6, **dadurch gekennzeichnet, dass** die Milchsäurebakterien Bakterien sind, welche in der Lage sind, an den menschlichen Intestinal-Zellen, unter Ausschluss pathogener Bakterien auf/unter den menschlichen Intestinal-Zellen, anzuhaften und/oder auf das menschliche Immunsystem einzuwirken, und ihm zu ermöglichen, auf externe Angriffe stärker zu reagieren.

8. Verfahren zum Herstellen eines Desserts gemäß einem der Ansprüche 1 bis 7, wobei eine Komposition, welche die Milchsäurebakterien enthält, und ein die Fasern enthaltender Träger separat präpariert werden, und das Dessert in der Weise gefroren wird, dass die genannten Bakterien im Wesentlichen nicht mit den genannten Fasern im Kontakt sind.

9. Verwendung einer gefrorenen Komposition gemäß einem der Ansprüche 1 bis 7, um ein zum Behandeln und/oder zur Prävention von gastrointestinalen Störungen vorgesehenes Diät-Produkt zu erhalten, um das menschliche Immunsystem zu unterstützen, oder um die Mineralien-Aufnahme zu steigern.

## Claims

1. Composite frozen dessert consisting of:
- an ice-cream containing probiotic lactic acid bacteria and
- an edible base substantially devoid of lactic acid bacteria,
in which the base is a covering or a bread or pastry article and comprises fermentable fibre that specifically favours the growth, in the intestinal tract, of the lactic acid bacteria present in the ice-cream.

2. Frozen dessert according to Claim 1, **characterized in that** the fibre is selected from vegetable pectins; chito-, fructo-, gentio-, galacto-, isomalto-, manno- and xylo-oligosaccharides; oligosaccharides of soya, *Polymnia sonchifolia*, artichoke, onion and asparagus; resistant starches; and products rich in β-glucans.

3. Frozen dessert according to Claim 1, **characterized in that** the ice-cream comprises more than 10⁶ cfu/g of lactic acid bacteria.

4. Frozen dessert according to Claim 1, **characterized in that** the ice-cream comprises encapsulated lactic acid bacteria.

5. Frozen dessert according to Claim 1, **characterized in that** the edible base is a covering of the non-bulked ice-cream type or a bread or pastry article.

6. Frozen dessert according to Claim 1, **characterized in that** the lactic acid bacteria are selected from acidophilic bacteria, Bifidobacteria and the species *Lactococcus lactis, Lactobacillus rhamnosus, Lactobacillus fermentum,* Lactobacillus *brevis, Lactobacillus plantarum, Lactobacillus casei, Lactobacillus delbruckii sbp. bulgaricus, Lactobacillus delbruckii sbp. lactis, Streptococcus thermophilus* and *Leuconostoc mesenteroides.*

7. Frozen dessert according to Claim 6, **characterized in that** the lactic acid bacteria are bacteria that are capable of adhering to human intestinal cells, of excluding pathogenic bacteria on human intestinal cells and/or of acting on the human immune system by enabling it to react more strongly to external aggressions.

8. Process for the manufacture of a dessert according to one of Claims 1 to 7 in which a composition containing the lactic acid bacteria, and a base containing the fibre, are prepared separately and the dessert is frozen in such a way that said bacteria are not substantially in contact with said fibre.

9. Use of a composite frozen composition according to one of Claims 1 to 7 for obtaining a dietetic product intended for the treatment and/or prevention of gastrointestinal disorders, for boosting the human immune system or for increasing the absorption of minerals.
